(19) Europäisches Patentamt
European Patent Office
Office européen des brevets

(11) Numéro de publication: **0 319 027 B1**

(12) **FASCICULE DE BREVET EUROPEEN**

(45) Date de publication de fascicule du brevet: **25.03.92**

(51) Int. Cl.$^5$: **A61K 7/06**

(21) Numéro de dépôt: **88120161.0**

(22) Date de dépôt: **02.12.88**

(54) **Composition pour induire et stimuler la croissance des cheveux et/ou freiner leur chute, à base d'un tensio-actif non ionique et d'un dérivé de pyrimidine.**

(30) Priorité: **04.12.87 LU 87067**

(43) Date de publication de la demande:
**07.06.89 Bulletin 89/23**

(45) Mention de la délivrance du brevet:
**25.03.92 Bulletin 92/13**

(84) Etats contractants désignés:
**AT BE CH DE FR GB IT LI NL SE**

(56) Documents cités:
**EP-A- 0 242 967**
**WO-A-85/04577**
**WO-A-88/01863**
**DE-A- 3 601 739**

(73) Titulaire: **L'OREAL**
**14, Rue Royale**
**F-75008 Paris(FR)**

(72) Inventeur: **Grollier, Jean François**
**16 bis Boulevard Morland**
**F-75004 Paris(FR)**

(74) Mandataire: **Casalonga, Axel et al**
**BUREAU D.A. CASALONGA - JOSSE Morassistrasse 8**
**W-8000 München 5(DE)**

**EP 0 319 027 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet une composition pour induire et stimuler la croissance des cheveux et/ou freiner leur chute contenant, en association, au moins un dérivé de pyrimidine et au moins un tensio-actif non ionique.

L'homme a un capital de 100.000 à 150.000 cheveux et il est normal de perdre quotidiennement 50 à 100 cheveux. La maintenance de ce capital résulte essentiellement du fait que la vie d'un cheveu est soumise à un cycle pilaire au cours duquel le cheveu se forme, croît et tombe, avant d'être remplacé par un nouveau cheveu qui apparaît dans le même follicule.

On observe au cours d'un cycle pilaire successivement trois phases, à savoir : la phase anagène, la phase catagène et la phase télogène.

Au cours de la première phase, dite anagène, le cheveu passe par une période de croissance active associée à une intense activité métabolique au niveau du bulbe.

La deuxième phase, dite catagène, est transitoire et elle est marquée par un ralentissement des activités mitotiques. Au cours de cette phase, le cheveu subit une involution, le follicule s'atrophie et son implantation dermique apparaît de plus en plus haute.

La phase terminale, dite télogène, correspond à une période de repos du follicule et le cheveu finit par tomber, poussé par un cheveu naissant en phase anagène.

Ce processus de renouvellement physique permanent subit une évolution naturelle au cours du vieillissement, les cheveux deviennent plus fins, leurs cycles plus courts.

L'alopécie survient lorsque ce processus de renouvellement physique est accéléré ou perturbé, c'est-à-dire que les phases de croissance sont raccourcies, le passage des cheveux en phase télogène est plus précoce et les cheveux tombent en plus grand nombre. Les cycles de croissance successifs aboutissent à des cheveux de plus en plus fins et de plus en plus courts, se transformant peu à peu en un duvet non pigmenté. Ce phénomène peut conduire à la calvitie.

Le cycle pilaire est tributaire de nombreux facteurs pouvant entraîner une alopécie plus ou moins prononcée. Parmi ces facteurs, on peut citer les facteurs alimentaires, endocriniens, nerveux, etc.

On détermine la variation de ces différentes catégories de cheveux grâce au trichogramme ou au phototrichogramme.

On recherche depuis de nombreuses années dans l'industrie cosmétique ou pharmaceutique des compositions permettant de supprimer ou de réduire l'alopécie et notamment d'induire ou de stimuler la croissance des cheveux ou de diminuer leur chute.

Dans cette optique, on a déjà proposé des composés tels que l'amino-6 dihydro-1,2 hydroxy-1 imino-2 pipéridino-4 pyrimidine et ses dérivés. De tels composés sont décrits notamment dans le brevet US-4.139.619.

Pour augmenter l'efficacité des compositions contenant les dérivés pyrimidiques, il s'est avéré intéressant de rechercher une composition bien tolérée contenant le(s) dérivé(s) de pyrimidine qui soit susceptible de rester en contact avec le scalp sans être rincée pendant une durée prolongée afin de favoriser la pénétration de la substance active dans la couche cornée.

Dans cette optique, la demanderesse a découvert que des compositions contenant certains tensio-actifs particuliers permettaient d'améliorer l'efficacité des compositions à base de(s) dérivé(s) de pyrimidine.

L'association de ces deux composés permet notamment d'obtenir par rapport à celles antérieurement connues, des compositions plus efficaces, ne nécessitant pas de rinçage pendant une durée prolongée, et très bien tolérées. Elle permet en outre de réaliser de nouvelles formes galéniques particulièrement bien adaptées à l'application du principe actif et à la possibilité de procéder à une élimination remarquablement aisée par simple rinçage à l'eau sur les cheveux et le cuir chevelu.

Grâce à la plus grande efficacité des compositions contenant l'association selon l'invention, on peut obtenir par rapport aux compositions de l'art antérieur, pour une même concentration en substance active, une activité plus importante et pour une concentration en substance active plus faible, une activité plus rapide.

La demanderesse a découvert en outre que contrairement à d'autres compositions associant le(s) dérivé(s) de pyrimidine avec des tensio-actifs classiquement utilisés dans le domaine de la cosmétique, les compositions proposées selon la présente invention présentaient une excellente stabilité dans le temps sans diminution de concentration en substance active et qu'elles ne présentaient aucune irritation du cuir chevelu après un contact prolongé de plusieurs heures sans être rincées.

La présente invention a donc pour objet une nouvelle composition pour induire et stimuler la croissance des cheveux et/ou freiner leur chute, contenant, dans un milieu physiologiquement acceptable, au moins un tensio-actif non ionique choisi parmi les poly(hydroxypropyléthers), les alkylphénols oxyéthylénés et au

moins un composé répondant à la formule :

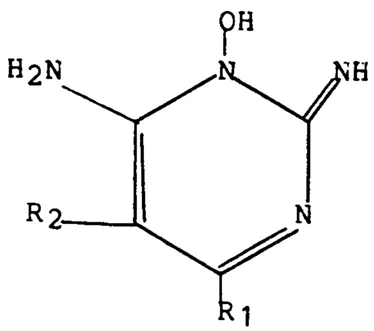

(I)

dans laquelle $R_1$ désigne un groupement

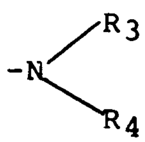

dans lequel :

$R_3$ et $R_4$ peuvent être choisis parmi :

l'hydrogène, un groupement alkyle, de préférence ayant 1 à 4 atomes de carbone, alcényle, alkylaryle ou cycloalkyle inférieur,

$R_3$ et $R_4$ peuvent également former un hétérocycle avec l'atome d'azote auquel ils sont liés, cet hétérocycle étant choisi parmi les groupements aziridinyle, azétidinyle, pyrrolidinyle, pipéridinyle, hexahydroazépinyle, heptaméthylèneimine, octaméthylèneimine, morpholine et alkyle inférieur-4-pipérazidinyle, les groupements hétérocycliques pouvant être substitués sur les atomes de carbone par un à trois groupements alkyles inférieur, hydroxy ou alcoxy;

le groupement $R_2$ est choisi parmi l'hydrogène, un groupement alkyle, alcényle, alkylalcoxy, cycloalkyle, aryle, alkylaryle, arylalkyle, alkylarylalkyle, alcoxyarylalkyle et haloarylalkyle inférieur, ainsi que les sels d'addition d'acides cosmétiquement ou pharmaceutiquement acceptables.

Dans la formule (I), alkyle ou alcoxy désigne de préférence un groupement ayant 1 à 4 atomes de carbone, alcényle désigne de préférence un groupement ayant 2 à 5 atomes de carbone et aryle désigne de préférence phényle.

Les composés de formule (I) plus particulièrement préférés sont choisis parmi les composés dans lesquels $R_2$ désigne hydrogène, et

$R_1$ représente un groupement

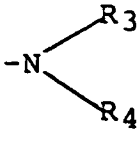

,

dans lequel $R_3$ et

$R_4$ forment un cycle pipéridinyle ainsi que leurs sels tel que par exemple le sulfate.

Le composé plus particulièrement préféré est l'amino-6 dihydro-1,2 hydroxy-1 imino-2 pipéridino-4 pyrimidine ou "Minoxidil".

Les tensio-actifs non ioniques poly(hydroxypropyléther) utilisés plus particulièrement selon l'invention sont choisis parmi les composés de formules (II) à (IV) ci-après et/ou parmi les composés préparés selon les procédés décrits dans les paragraphes (3) et (4) ci-dessous :

$$(1)\quad R_5O-(CH_2-\underset{\displaystyle CH_2OH}{\underset{|}{CH}}O)_m-H \qquad (II)$$

où $R_5$ désigne un radical ou un mélange de radicaux alkyles contenant 10 à 14 atomes de carbone et m est un nombre entier ou décimal de 2 à 10 et de préférence de 3 à 6. Ces composés de formule (II) peuvent être préparés selon le procédé décrit dans le brevet FR-1.477.048;

$$(2)\quad R_6-CONH-CH_2-CH_2-O-CH_2-CH_2-O(CH_2-CHOH-CH_2-O)_n-H \qquad (III)$$

où $R_6$ désigne un radical ou un mélange de radicaux alkyles et/ou alcényles ayant de 11 à 17 atomes de carbone, et n désigne un nombre entier ou décimal de 1 à 5 et de préférence 1,5 à 4.

Ces composés de formule (III) peuvent être préparés selon le procédé décrit dans le brevet FR-2.328.763;

$$(3)\ \text{composés de formule}\ R_7CHOH-CH_2-O(CH_2-CHOH-CH_2-O)_p-H \qquad (IV)$$

dans laquelle $R_7$ désigne un radical aliphatique, cycloaliphatique, arylaliphatique, ayant de préférence 7 à 21 atomes de carbone, et leurs mélanges, les chaînes aliphatiques désignant en particulier des chaînes alkyle pouvant comporter 1 à 6 groupements éther, thioéther et/ou hydroxyméthylène et p est un nombre compris entre 1 et 10 inclus.

Ces composés sont préparés par condensation en catalyse alcaline de 2 à 10 et de préférence de 2,5 à 6 moles de glycidol sur un alpha-diol ou un mélange d'alpha-diols en $C_{10}$-$C_{14}$ à la température de 120-180 °C et de préférence de 140 à 160 °C, le glycidol étant ajouté lentement selon le procédé de préparation décrit dans le brevet FR-2.091.516;

(4) composés préparés par condensation, en catalyse acide, de 2 à 10 et de préférence de 2,5 à 6 moles de glycidol par mole d'alcool ou d'alpha-diol contenant 10 à 14 atomes de carbone à une température de 50 à 120 °C, le glycidol étant ajouté lentement à l'alcool ou à l'alpha-diol, le procédé de préparation de ces composés étant décrit dans le brevet FR-2.169.787;

(5) composés de poly(hydroxypropyléther) préparés par (poly)addition de monochlorhydrine du glycérol sur un composé organique (poly)hydroxylé en présence d'une base forte, par élimination au fur et à mesure de l'eau par distillation, décrits en particulier dans le brevet français FR-2.574.786.

Parmi les tensio-actifs non ioniques poly (hydroxypropyléther) décrits sous les paragraphes (1), (2), (3), (4) et (5) ci-dessus, les composés ci-après sont préférés :

$$C_{12}H_{25}O-(CH_2-\underset{\displaystyle CH_2OH}{\underset{|}{CH}}-O)_{4,2}-H \qquad (V)$$

$$R_5O-(CH_2-\underset{\displaystyle CH_2OH}{\underset{|}{CH}}-O)_{3,75}-H \qquad (VI)$$

où $R_5$ désigne un mélange de radicaux alkyle $C_{10}H_{21}$ et $C_{12}H_{25}$;

- les composés préparés par condensation en catalyse alcaline de 3,5 moles de glycidol sur un mélange d'alpha-diols ayant de 11 à 14 atomes de carbone, selon le procédé décrit dans le brevet FR-2.091.516;
- et les composés de formule :

$$R_6\text{-CONH-CH}_2\text{-CH}_2\text{-O-CH}_2\text{-CH}_2\text{-O-}(\text{CH}_2\text{-CHOH-CH}_2\text{-O})_{3,5}\text{H} \quad (VII)$$

où $R_6$ désigne un mélange de radicaux comprenant les radicaux alkyles et alkényles suivants : $C_{11}H_{23}$, $C_{13}H_{27}$, radicaux dérivés des acides gras du coprah, radical dérivé de l'acide oléïque;

- les composés préparés par condensation de 3,5 moles de glycidol sur un mélange d'alpha-diols en $C_{11}$-$C_{14}$, selon le brevet FR-2.091.516, le tensio-actif non ionique poly(hydroxypropyléther) obtenu par condensation de la monochlorhydrine (2,5 moles) en présence de soude sur le dodécanediol-1,2, sont particulièrement préférés.

Les alkylphénols oxyéthylénés comportent 1 à 100 moles d'oxyde d'éthylène et le radical alkyle comporte de préférence 8 ou 9 atomes de carbone dans la chaîne. On peut peut citer en particulier :

- les octylphénol oxyéthylénés, comportant de 8 à 70 moles d'oxyde d'éthylène, tels que par exemple et sans limitation, les produits vendus sous les dénominations :
  . SINNOPAL® OP par la Société HENKEL
  . TRITON® X par la Société ROHM & HAAS
  . IGEPAL® CA par la Société GAF
  . ANTAROX® CA 630 par la Société GAF
- les nonylphénol oxyéthylénés, comportant de 1 à 100 moles d'oxyde d'éthylène, tels que par exemple et sans limitation, les produits vendus sous les dénominations :
  . SINNOPAL® NP par la Société HENKEL
  . TRITON® N par la Société ROHM & HAAS
  . IGEPAL® CO par la Société GAF
  . NEUTRONYX® par la Société ONYX
  . CARSONON® N par la Société LONZA
  . SURFONIC® N par la Société TEXACO
  . ANTAROX® CO par la Société GAF

  et plus particulièrement les alkyls $C_8$-$C_9$ phénols oxyéthylénés par 5 à 35 moles d'oxyde d'éthylène.

Une composition plus particulièrement préférée, conforme à l'invention, est constituée par une composition contenant, dans un milieu cosmétiquement ou pharmaceutiquement acceptable, au moins l'amino-6 dihydro-1,2 hydroxy-1 imino-2 pipéridino-4 pyrimidine et un tensio-actif non ionique poly-(hydroxypropyléther) défini ci-dessus.

Le dérivé de pyrimidine de formule I est utilisé dans les compositions conformes à l'invention dans une proportion de 0,05 à 6% en poids par rapport au poids total de la composition, et de préférence de 0,1 à 3% et plus particulièrement de 0,5 à 2%.

Le tensio-actif non ionique est utilisé dans des proportions comprises entre 1 et 30% en poids par rapport au poids total de la composition et de préférence de 5 à 15%.

Le milieu cosmétiquement ou pharmaceutiquement acceptable peut être un milieu aqueux ou un mélange d'eau et d'un solvant cosmétiquement ou pharmaceutiquement acceptable épaissi ou non.

A titre de solvant on peut utiliser notamment des alcools inférieurs en $C_{1-4}$ tels que l'alcool éthylique, l'alcool isopropylique et l'alcool tert.-butylique, des alkylène-glycols comme le propylèneglycol et des alkyléthers de mono- et de dialkylèneglycols tels que le monoéthyléther d'éthylèneglycol, le monométhyléther de propylèneglycol et le monoéthyléther de diéthylèneglycol.

A titre d'épaississants, on peut utiliser les agents épaississants bien connus dans l'état de la technique dont on peut citer plus particulièrement, à titre d'exemple non limitatif, les hétérobiopolysaccharides tels que la gomme de xanthane ou les scléroglucanes, les dérivés de cellulose, les polymères acryliques réticulés ou non.

Ces épaississants sont utilisés de préférence dans des proportions comprises entre 0,1 et 5% en poids par rapport au poids total de la composition.

Ces compositions peuvent éventuellement renfermer les ingrédients classiquement utilisés dans des compositions cosmétiques ou pharmaceutiques tels que des agents conservateurs, des agents complexants, des colorants, des agents alcalinisants ou acidifiants, des parfums, des polymères.

Le pH de ces compositions est compris entre 4 et 9 et de préférence entre 7 et 8,5.

Elles peuvent se présenter sous forme de lotions épaissies ou non, de gels, de crèmes, d'émulsions et être pressurisées dans des dispositifs aérosols.

Le procédé de traitement pour lutter contre la chute des cheveux et/ou stimuler leur croissance consiste principalement à appliquer sur les zones alopéciques du cuir chevelu et les cheveux d'un individu une

composition telle que définie ci-dessus, à laisser en contact plusieurs heures et éventuellement à rincer. On peut par exemple appliquer la composition sur les cheveux et le cuir chevelu le soir, garder la composition toute la nuit et éventuellement effectuer un shampooing le matin ou laver les cheveux à l'aide de ladite composition et laisser à nouveau en contact quelques minutes avant de rincer.

Le procédé conforme à l'invention présente les caractéristiques d'un traitement thérapeutique des alopécies dans la mesure où la composition agit sur les mécanismes biologiques et notamment sur le cycle pilaire.

Il présente également des caractéristiques d'un traitement cosmétique dans la mesure où il permet d'embellir les cheveux et le cuir chevelu.

Les exemples suivants sont destinés à illustrer l'invention sans pour autant présenter un caractère limitatif.

EXEMPLE 1

On prépare la composition suivante :

| | | |
|---|---|---|
| - Amino-6 dihydro-1,2 hydroxy-1 imino-2 pipéridino-4 pyrimidine | | 0,50 g |
| - Gomme de xanthane vendue sous la dénomination "KELZAN® K9 C57" par la Société KELCO | | 1,00 g |
| - Tensio-actif non ionique poly(hydroxy-propyléther) préparé par condensation en catalyse alcaline de 3,5 moles de glycidol sur un mélange d'alpha-diols ayant de 11 à 14 atomes de carbone, selon le procédé décrit dans le brevet FR-2.091.516 | | 10,00 g |
| - Eau | qsp | 100,00 g |

EXEMPLE 2

On prépare la lotion de composition suivante :

| | |
|---|---|
| - Amino-6 dihydro-1,2 hydroxy-1 imino-2 pipéridino-4 pyrimidine | 0,25 g |
| - Tensio-actif non ionique poly (hydroxy-propyléther) de formule : | |

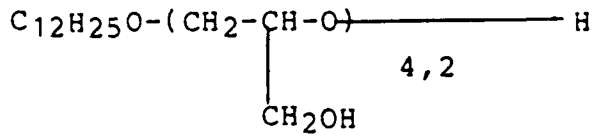 5,00 g

- Eau qsp 100,00 g

EXEMPLE 3

On prépare la lotion de composition suivante :

| | | |
|---|---|---|
| - Amino-6 dihydro-1,2 hydroxy-1 imino-2 pipéridino-4 pyrimidine | | 0,55 g |
| - Tensio-actif non ionique poly(hydroxypropyléther) de formule : $R-CONH-(CH_2)_2-O-(CH_2)_2-O-(CH_2-CHOH-CH_2-O)_{3,5}-H$ où R désigne le mélange suivant de radicaux alkyles et alcényles : 35% $C_{12}H_{25}$ - 15% $C_{14}H_{29}$ - 15% radicaux oléyle - 35% radicaux dérivés des acides gras du coprah | | 12,00 g |
| - Eau | qsp | 100,00 g |

EXEMPLE 4

On prépare la lotion de composition suivante :

| | | |
|---|---|---|
| - Amino-6 dihydro-1,2 hydroxy-1 imino-2 pipéridino-4 pyrimidine | | 2,00 g |
| - Tensio-actif non ionique poly(hydroxypropyléther) obtenu par condensation de la monochlorhydrine du glycérol (2,5 moles) en présence de soude sur le dodécanediol-1,2 selon le brevet français FR-25.74.786 | | 10,00 g |
| - Propylèneglycol | | 20,00 g |
| - Alcool éthylique | | 50,00 g |
| - Eau | qsp | 100,00 g |

On applique cette composition sur le cuir chevelu et les cheveux le soir à raison de 15 g, à la fréquence hebdomadaire de une à trois fois. On laisse poser 5 à 30 minutes, puis on rince à l'eau.

EXEMPLES 5 à 7

On prépare les lotions de composition suivante :

| | EX. 5 | EX. 6 | EX. 7 |
|---|---|---|---|
| Amino-6 dihydro-1,2 hydroxy-1 imino-2 pipéridino-4 pyrimidine | 0,25 g | 0,25 g | 0,25 g |
| Nonylphénol oxyéthyléné à 10 moles d'oxyde d'éthylène ANTAROX® CO 630 (GAF) | 15 g | | |
| Nonylphénol oxyéthyléné à 16 moles d'oxyde d'éthylène ANTAROX® CO 730 (GAF) | | 15 g | |
| Nonylphénol oxyéthyléné à 31 moles d'oxyde d'éthylène ANTAROX® CO 880 (GAF) | | | 15 g |
| Sel disodique de l'acide éthylène diamino tétracétique ($Na_2EDTA$) | 0,2 g | 0,2 g | 0,2 g |
| Eau qsp | 100 g | 100 g | 100 g |

Les lotions des exemples 1 à 7 présentent une excellente stabilité dans le temps sans perte de principe actif.

Les compositions des exemples 1 à 7 ont été appliquées sur le cuir chevelu à raison de 13 g sur une surface de 100 $cm^2$ . On constate que la composition ne coule pas et s'élimine facilement au rinçage en laissant les cheveux brillants et légers.

On applique ces compositions pendant quelques mois à raison d'une application le soir, une à trois fois par semaine, et après chaque application, on rince le matin.

On constate une augmentation du rapport

$$\frac{A}{T}\left(\frac{\text{Anagène}}{\text{Télogène}}\right)$$

## Revendications

**1.** Composition pour induire et stimuler la croissance des cheveux et/ou freiner leur chute, caractérisée par le fait qu'elle contient dans un milieu cosmétiquement ou pharmaceutiquement acceptable, au moins un tensio-actif non ionique choisi parmi les poly(hydroxypropyléthers), les alkylphénols oxyéthylénés et au moins un composé répondant à la formule :

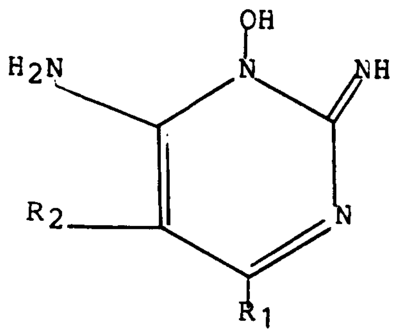

(I)

dans laquelle $R_1$ désigne un groupement

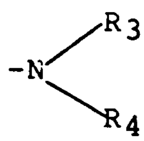

dans lequel :

$R_3$ et $R_4$ peuvent être choisis parmi l'hydrogène, un groupement alkyle, alcényle, alkylaryle ou cycloalkyle, $R_3$ et $R_4$ peuvent également former un hétérocycle avec l'atome d'azote auquel ils sont liés, cet hétérocycle étant choisi parmi les groupements aziridinyle, azétidinyle, pyrrolidinyle, pipéridinyle, hexahydroazépinyle, heptaméthylèneimine, octaméthylèneimine, morpholine et alkyle inférieur-4-pipérazidinyle, les groupements hétérocycliques pouvant être substitués sur les atomes de carbone par un à trois groupements alkyle, hydroxy ou alcoxy;

le groupement $R_2$ est choisi parmi l'hydrogène, un groupement alkyle, alcényle, alkylalcoxy, cycloalkyle, aryle, alkylaryle, arylalkyle, alkylarylalkyle, alcoxyarylalkyle et/ou haloarylalkyle, ainsi que les sels d'addition d'acides cosmétiquement ou pharmaceutiquement acceptables.

2. Composition selon la revendication 1, caractérisée par le fait que le composé (I) est un composé dans lequel $R_2$ désigne l'hydrogène et $R_1$ représente le groupement

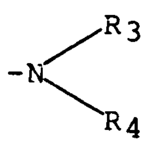 ,

dans lequel $R_3$ et $R_4$ forment un cycle pipéridinyle ou l'un de ses sels.

3. Composition selon la revendication 1, caractérisée par le fait que le composé de formule (I) est l'amino-6 dihydro-1,2 hydroxy-1 imino-2 pipéridino-4 pyrimidine ou "minoxidil".

4. Composition selon l'une quelconque des revendications 1 à 3, caractérisée par le fait que le tensio-actif non ionique est un poly(hydroxypropyléther) choisi dans le groupe formé par :

(1) les composés de formule (II) :

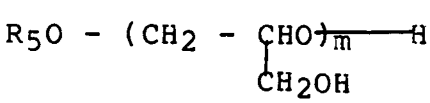

(II)

où $R_5$ désigne un radical ou un mélange de radicaux alkyles contenant 10 à 14 atomes de carbone et m est un nombre entier ou décimal de 2 à 10 et de préférence de 3 à 6;

(2) les composés de formule (III) :

$$R_6\text{-CONH-CH}_2\text{-CH}_2\text{-O-CH}_2\text{-CH}_2\text{-O}(\text{CH}_2\text{-CHOH-CH}_2\text{-O})_n\text{-H} \quad \text{(III)}$$

où $R_6$ désigne un radical ou un mélange de radicaux alkyles et/ou alkényles, ayant de 11 à 17 atomes de carbone et n désigne un nombre entier ou décimal de 1 à 5 et de préférence de 1,5 à 4;
(3) les composés de formule (IV) :

$$R_7\text{CHOH-CH}_2\text{-O}(\text{CH}_2\text{-CHOH-CH}_2\text{-O})_p\text{-H} \quad \text{(IV)}$$

dans laquelle $R_7$ désigne un radical aliphatique, cycloaliphatique, arylaliphatique, ayant de préférence 7 à 21 atomes de carbone, et leurs mélanges, les chaînes aliphatiques désignant en particulier des chaînes alkyle pouvant comporter 1 à 6 groupements éther, thioéther et/ou hydroxyméthylène et p est un nombre compris entre 1 et 10 inclus;
(4) les composés préparés par condensation en catalyse acide, de 2 à 10 et de préférence de 2,5 à 6 moles de glycidol par mole d'alcool ou d'alpha-diol contenant 10 à 14 atomes de carbone;
(5) les composés de poly(hydroxypropyléther) préparés par (poly)addition de monochlorhydrine de glycérol sur un composé organique poly(hydroxylé) en présence d'une base forte et élimination d'eau.

**5.** Composition selon la revendication 4, caractérisée par le fait que le tensio-actif non ionique poly-(hydroxypropyléther) est choisi parmi les composés de formules :

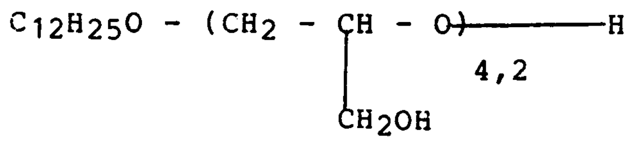

( V)

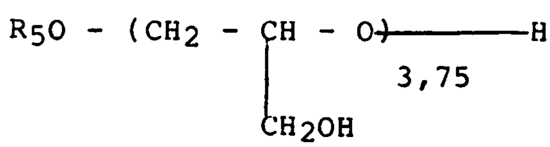

(VI)

ou $R_5$ designe un melange de radicaux alkyle $C_{10}H_{21}$ et $C_{12}H_{25}$.

**6.** Composition selon la revendication 4, caractérisée par le fait que le tensio-actif non ionique de poly-(hydroxypropyléther) est le composé :

(VII)

$$R_6\text{-CONH-CH}_2\text{-CH}_2\text{-O-CH}_2\text{-CH}_2\text{-O-}(\text{CH}_2\text{-CHOH-CH}_2\text{-O})_{3,5}\text{-H}$$

où $R_6$ désigne un mélange de radicaux comprenant les radicaux alkyle et alkényle suivants : $C_{11}H_{23}$, $C_{13}H_{27}$, radicaux dérivés des acides gras du coprah, radical dérivé de l'acide oléïque.

**7.** Composition selon la revendication 4, caractérisée par le fait que le tensio-actif non ionique poly-(hydroxypropyléther) est préparé par condensation en catalyse alcaline de 3,5 moles de glycidol sur un mélange d'alpha-diols ayant de 11 à 14 atomes de carbone.

**8.** Composition selon la revendication 4, caractérisée par le fait que le composé de poly-(hydroxypropyléther) (5) est obtenu par condensation de la monochlorhydrine (2,5 moles) en présence de soude sur le dodécanediol-1,2.

9. Composition selon l'une quelconque des revendications 1 à 3, caractérisée par le fait que le tensio-actif non ionique est un alkyl $C_8$-$C_9$ phénol oxyéthyléné avec 1 à 100 moles d'oxyde d'éthylène.

10. Composition selon la revendication 1, caractérisée par le fait qu'elle contient l'amino-6 dihydro-1,2 hydroxy-1 imino-2 pipéridino-4 pyrimidine et un tensio-actif non ionique de poly(hydroxypropyléther).

11. Composition selon l'une quelconque des revendications 1 à 10, caractérisée par le fait que le composé de formule (I) est présent dans des proportions comprises entre 0,05 et 6% en poids par rapport au poids total de la composition et de préférence entre 0,1 et 3% en poids.

12. Composition selon l'une quelconque des revendications 1 à 11, caractérisée par le fait que le tensio-actif non ionique est présent dans des proportions comprises entre 1 et 30% en poids par rapport au poids total de la composition et de préférence entre 5 et 15% en poids.

13. Composition selon l'une quelconque des revendications 1 à 12, caractérisée par le fait que le milieu cosmétiquement ou pharmaceutiquement acceptable est constitué par de l'eau ou un mélange d'eau et d'un solvant cosmétiquement ou pharmaceutiquement acceptable, épaissi ou non.

14. Composition selon la revendication 13, caractérisée par le fait qu'elle contient des agents épaississants dans des proportions comprises entre 0,1 et 5% en poids par rapport au poids total de la composition.

15. Procédé de traitement cosmétique des cheveux, caractérisé par le fait que l'on applique sur les cheveux ou le cuir chevelu au moins une composition telle que définie dans l'une quelconque des revendications 1 à 14 que l'on laisse en contact et qu'éventuellement, on rince.

16. Composition selon l'une quelconque des revendications 1 à 14 pour son application dans le traitement thérapeutique de l'alopécie.

17. Utilisation de la composition selon l'une quelconque des revendication 1 à 14 et 16 pour la préparation d'un médicament destiné au traitement de l'alopécie.

**Claims**

1. Composition for inducing and stimulating the growth of hair and/or for slowing down its loss, characterised in that it contains in a cosmetically or pharmaceutically acceptable medium at least one nonionic surface-active agent chosen from poly(hydroxypropyl ethers), oxyethylenated alkylphenols and at least one compound of the formula:

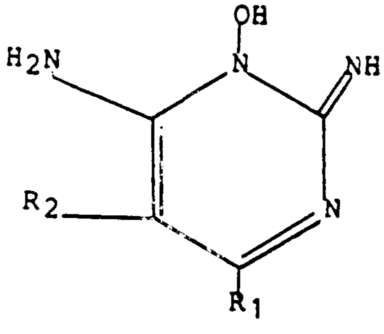

(I)

in which $R_1$ denotes a group

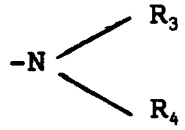

in which:
$R_3$ and $R_4$ may be chosen from hydrogen, an alkyl, an alkenyl, an alkylaryl or a cycloalkyl group, $R_3$ and $R_4$ may also form a heterocycle with the nitrogen atom to which they are attached, this heterocycle being chosen from the aziridinyl, azetidinyl, pyrrolidinyl, piperidinyl, hexahydroazepinyl, heptamethyleneimine, octamethyleneimine, morpholine and 4-lower allkylpiperazidinyl groups, it being possible for the heterocyclic groups to be substituted on the carbon atoms by one to three alkyl, hydroxy or alkoxy groups;
the group $R_2$ is chosen from hydrogen, an alkyl, an alkenyl, an alkylalkoxy, a cycloalkyl, an aryl, an alkylaryl, an arylalkyl, an alkylarylalkyl, an alkoxyarylalkyl and/or a haloarylalkyl group as well as the addition salts of cosmetically or pharmaceutically acceptable acids.

**2.** Composition according to Claim 1, characterised in that the compound (I) is a compound in which $R_2$ denotes hydrogen and $R_1$ represents the group

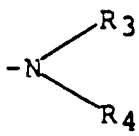 ,

in which $R_3$ and $R_4$ form a piperidinyl ring or one of its salts.

**3.** Composition according to Claim 1, characterised in that the compound of formula (I) is 6-amino-1,2-dihydro-1-hydroxy-2-imino-4-piperidinylpyrimidine or "minoxidil".

**4.** Composition according to any one of Claims 1 to 3, characterised in that the nonionic surface-active agent is a poly(hydroxypropylether) chosen from the group formed by:
(1) the compounds of formula (II):

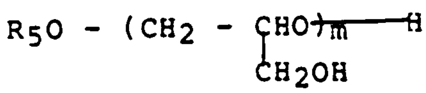 (II)

where $R_5$ denotes an alkyl radical or mixture of radicals containing 10 to 14 carbon atoms and m is an integer or a decimal from 2 to 10 and preferably from 3 to 6;
(2) the compounds of formula (III):

$$R_6-CONH-CH_2-CH_2-O-CH_2-CH_2-O(CH_2-CHOH-CH_2-O)_n-H \quad (III)$$

where $R_6$ denotes an alkyl and/or an alkenyl radical or mixture of radicals having from 11 to 17 carbon atoms and n denotes an integer or a decimal from 1 to 5 and preferably from 1.5 to 4;
(3) the compounds of formula (IV):

$$R_7CHOH-CH_2-O(CH_2-CHOH-CH_2-O)_p-H \quad (IV)$$

in which $R_7$ denotes an aliphatic, a cycloaliphatic, or an arylaliphatic radical preferably having 7 to 21 carbon atoms, and their mixtures, the aliphatic chains denoting in particular alkyl chains which may comprise 1 to 6 ether, thioether and/or hydroxymethylene groups and p is a number between 1 and 10 inclusive;
(4) the compounds prepared by condensation using acid catalysis, of 2 to 10 and preferably of 2.5 to 6 mols of glycidol per mole of alcohol or alpha-diol containing 10 to 14 carbon atoms;
(5) the compounds of poly(hydroxypropylether) prepared by poly-addition of glycerol monochlorohydrin with a polyhydroxylated organic compound in the presence of a strong base and elimination of water.

5. Composition according to Claim 4, characterised in that the poly(hydroxypropylether) nonionic surface-active agent is chosen from the compounds of formulae:

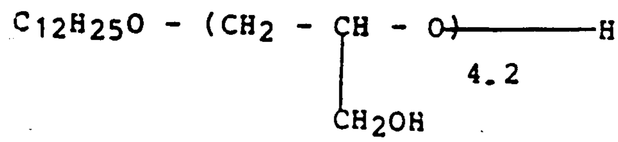

(V)

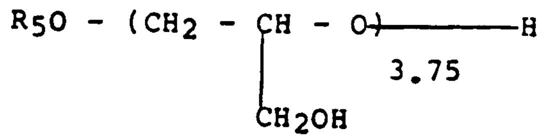

(VI)

where $R_5$ denotes a mixture of $C_{10}H_{21}$ and $C_{12}H_{25}$ alkyl radicals.

6. Composition according to Claim 4, characterised in that the poly(hydroxypropylether) nonionic surface-active agent is the compound:

$$R_6\text{-CONH-CH}_2\text{-CH}_2\text{-O-CH}_2\text{-CH}_2\text{-O-}(\text{CH}_2\text{-CHOH-CH}_2\text{-O})_{3.5}\text{-H} \quad \text{(VII)}$$

where $R_6$ denotes a mixture of radicals comprising the following alkyl and alkenyl radicals: $C_{11}H_{23}$, $C_{13}H_{27}$, radicals derived from the fatty acids of coprah, radical derived from oleic acid.

7. Composition according to Claim 4, characterised in that the poly(hydroxypropylether) nonionic surface-active agent is prepared by condensation using alkaline catalysis of 3.5 moles of glycidol with a mixture of alpha-diols having 11 to 14 carbon atoms.

8. Composition according to Claim 4, characterised in that the poly(hydroxypropylether) compound (5) is obtained by condensation of monochlorohydrin (2.5 moles) with 1,2-dodecanediol in the presence of sodium hydroxide.

9. Composition according to any one of Claims 1 to 3, characterised in that the nonionic surface-active agent is a $C_8$-$C_9$ alkylphenol oxyethylenated with 1 to 100 moles of ethylene oxide.

10. Composition according to Claim 1, characterised in that it contains 6-amino-1,2-dihydro-1-hydroxy-2-amino-4-piperidinylpyrimidine and a poly(hydroxypropylether) nonionic surface-active agent.

11. Composition according to any one of Claims 1 to 10, characterised in that the compound of formula (I) is present in proportions of between 0.05 and 6% by weight relative to the total weight of the composition and preferably between 0.1 and 3% by weight.

12. Composition according to any one of Claims 1 to 11, characterised in that the nonionic surface-active agent is present in proportions of between 1 and 30% by weight relative to the total weight of the composition and preferably between 5 and 15% by weight.

13. Composition according to any one of Claims 1 to 12, characterised in that the cosmetically or pharmaceutically acceptable medium consists of water or a mixture of water and a cosmetically or pharmaceutically acceptable solvent, thickened or unthickened.

14. Composition according to Claim 13, characterised in that it contains thickening agents in proportions of between 0.1 and 5% by weight relative to the total weight of the composition.

15. Process for the cosmetic treatment of hair, characterised in that there is applied to the hair or to the

scalp at least one composition such as defined in any one of Claims 1 to 14 which is left in contact and which is rinsed optionally.

**16.** Composition according to any one of Claims 1 to 14 to be applied in the therapeutic treatment of alopecia.

**17.** Use of the composition according to any one of Claims 1 to 14 and 16 for the preparation of a medicinal substance intended for the treatment of alopecia.

## Patentansprüche

**1.** Mittel zum Induzieren und Stimulieren des Haarwuchses und/oder zum Vermindern des Haarausfalls, dadurch **gekennzeichnet,** daß es in eniem kosmetisch oder pharmazeutisch verträglichen Milieu mindestens ein nicht-ionisches Tensid, ausgewählt unter Poly(hydroxypropylethern), oxethylierten Alkylphenolen, und mindestens eine Verbindung entsprechen

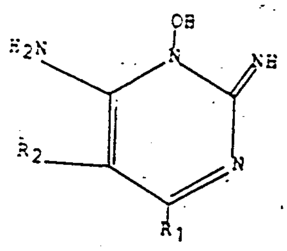

(I)

worin $R_1$ eine

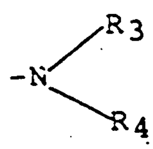

-Gruppe darstellt, worin $R_3$ und $R_4$ unter Wasserstoff, einer Alkyl-, Alkenyl-, Alkylaryl- oder Cycloalkylgruppe ausgewählt sein, und $R_3$ und $R_4$ auch einen Heterozyklus mit dem Stickstoffatom, an das sie gebunden sind, bilden können, wobei dieser Heterozyklus unter Aziridinyl-, Azetidinyl-, Pyrrolidinyl-, Piperidinyl-, Hexahydroazepinyl-, Heptamethylenimin-, Octamethylenimin-, Morpholin- und Niedrigalkyl-4-piperazidinylgruppen ausgewählt ist, wobei die heterozyklischen Gruppen an den Kohlenstoffatomen durch eine bis drei Alkyl-, Hydroxy- oder Alkoxygruppen substituiert sein können;
und die Gruppe $R_2$ unter Wasserstoff, einer Alkyl-, Alkenyl-, Alkylalkoxy-, Cycloalkyl-, Aryl-, Alkylaryl-, Arylalkyl-, Alkylarylalkyl-, Alkoxyarylalkyl- und/oder Haloarylalkylgruppe ausgewählt ist,
sowie die kosmetisch oder pharmazeutisch verträglichen Säureadditionssalze davon.

**2.** Mittel gemäß Anspruch 1,
dadurch **gekennzeichnet**, daß
die Verbindung (I) eine Verbindung, worin $R_2$ Wasserstoff bedeutet und $R_1$ die

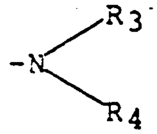

-Gruppe darstellt, worin $R_3$ und $R_4$ einen Piperidinring bilden,
oder ein Salz davon ist.

3. Mittel gemäß Anspruch 1,
dadurch **gekennzeichnet,** daß
die Verbindung der Formel (I) Amino-6-dihydro-1,2-hydroxy-1-imino-2-piperidino-4-pyrimidin oder "Minoxidil" ist.

4. Mittel gemäß eines jeden der Ansprüche 1 bis 3,
dadurch **gekennzeichnet,** daß
das nicht-ionische Tensid ein Poly(hydroxypropylether) ist, ausgewählt unter der Gruppe, gebildet aus:
(1) Verbindungen der Formel (II):

$$R_5O-(CH_2-\underset{\substack{|\\ CH_2OH}}{C}HO)_m-H \qquad (II)$$

worin $R_5$ einen Rest oder eine Mischung von Alkylresten mit 10 bis 14 Kohlenstoffatomen bezeichnet und m eine ganze Zahl oder eine Dezimalzahl von 2 bis 10 und vorzugsweise von 3 bis 6 ist;
(2) Verbindungen der Formel (III):

$$R_6-CONH-CH_2-CH_2-O-CH_2-CH_2-O(CH_2-CHOH-CH_2-O)_n-H \qquad (III)$$

worin $R_6$ ein Rest oder eine Mischung von Alkyl- und/oder Alkenylresten mit 11 bis 17 Kohlenstoffatomen bezeichnet und n eine ganze Zahl oder eine Dezimalzahl von 1 bis 5 und vorzugsweise von 1,5 bis 4 bedeutet;
(3) Verbindungen der Formel (IV):

$$R_7CHOH-CH_2-O(CH_2-CHOH-CH_2-O)_p-H \qquad (IV)$$

worin $R_7$ einen aliphatischen, cycloaliphatischen, arylaliphatischen Rest mit vorzugsweise 7 bis 21 Kohlenstoffatomen sowie deren Mischungen bedeutet, wobei die aliphatischen Ketten insbesondere Alkylketten darstellen, die 1 bis 6 Ether-, Thioether-und/oder Hydroxymethylengruppen aufweisen können, und p eine Zahl zwischen 1 und 10 ist;
(4) Verbindungen, hergestellt durch Kondensation unter sauerer Katalyse von 2 bis 10 und vorzugsweise von 2,5 bis 6 Mol Glycidol pro Mol Alkohol oder alpha-Diol mit 10 bis 14 Kohlenstoffatomen;
(5) Verbindungen von Poly(hydroxypropylether), hergestellt durch Polyaddition des Monochlorhydrins von Glycerin an eine organische polyhydroxilierte Verbindung in der Gegenwart einer starken Base unter Wasseraustritt.

5. Mittel gemäß Anspruch 4,
dadurch **gekennzeichnet,** daß
das nicht-ionische Tensid Poly(hydroxypropylether) unter Verbindungen der Formel ausgewählt ist:

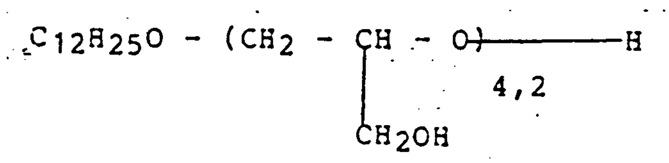 (V)

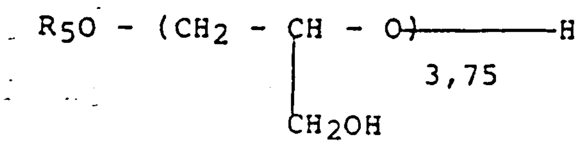 (VI)

worin $R_5$ eine Mischung von $C_{10}H_{21}$- und $C_{12}H_{25}$-Alkylresten bedeutet.

**6.** Mittel gemäß Anspruch 4,
dadurch **gekennzeichnet**, daß
das nicht-ionische Tensid des Poly(hydroxypropylethers) die Verbindung ist:

$$R_6-CONH-CH_2-CH_2-O-CH_2-CH_2-O-(CH_2-CHOH-CH_2-O)_{3,5}-H \quad (VII)$$

worin $R_6$ eine Mischung von Resten bedeutet, welche die folgenden Alkyl- und Alkenylreste aufweisen: $C_{11}H_{23}$, $C_{13}H_{27}$; von den Fettsäuren von Kopra abgeleitete Reste, einen von Ölsäure abgeleiteten Rest.

**7.** Mittel gemäß Anspuch 4,
dadurch **gekennzeichnet,** daß
das nicht-ionische Tensid Poly(hydroxypropylether) durch Kondensation unter alkalischer Katalyse von 3,5 Mol Glycidol an eine Mischung von alpha-Diolen mit 11 bis 14 Kohlenstoffatomen hergestellt ist.

**8.** Mittel gemäß Anspruch 4,
dadurch **gekennzeichnet,** daß
die Verbindung des Poly(hydroxypropylethers) (5) durch Kondensation des Monochlorhydrins (2,5 Mol) in Gegenwart von Soda an Dodecandiol-1,2 erhältlich ist.

**9.** Mittel gemäß eines jeden der Ansprüche 1 bis 3,
dadurch **gekennzeichnet,** daß
das nicht-ionische Tensid ein mit 1 bis 100 Mol Ethylenoxid oxethyliertes $C_8$-$C_9$-Alkylphenol ist.

**10.** Mittel gemäß Anspruch 1,
dadurch **gekennzeichnet,** daß
es Amino-6-dihydro-1,2-hydroxy-1-imino-2-piperidino-4-pyrimidin und ein nicht-ionisches Tensid von Poly(hydroxypropylether) enthält.

**11.** Mittel gemäß eines jeden der Ansprüche 1 bis 10,
dadurch **gekennzeichnet,** daß
die Verbindung der Formel (I) in Verhältnissen von 0,05 bis 6 Gew.%, bezogen auf Gesamtgewicht des Mittels, und vorzugsweise von 0,1 bis 3 Gew.%, vorliegt.

**12.** Mittel gemäß eines jeden der Ansprüche 1 bis 11,
dadurch **gekennzeichnet,** daß
das nicht-ionische Tensid in Verhältnissen von 1 bis 30 Gew.%, bezogen auf Gesamtgewicht des Mittels, und vorzugsweise von 5 bis 15 Gew.% vorliegt.

**13.** Mittel gemäß eines jeden der Ansprüche 1 bis 12,
dadurch **gekennzeichnet,** daß
das kosmetisch oder pharmazeutisch verträgliche Milieu aus Wasser oder einer Mischung aus Wasser und einem kosmetisch oder pharmazeutisch verträglichen Lösungsmittel, verdickt oder nicht, zusammengesetzt ist.

**14.** Mittel gemäß Anspruch 13,
dadurch **gekennzeichnet,** daß
es Verdickungsmittel in Verhältnissen von 0,1 bis 5 Gew.%, bezogen auf Gesamtgewicht des Mittels, enthält.

**15.** Verfahren zur kosmetischen Behandlung von Haaren,
dadurch **gekennzeichnet,** daß
man auf die Haare oder die behaarte Haut mindestens ein Mittel gemäß eines jeden der Ansprüche 1 bis 14 aufbringt, es in Kontakt läßt und gegebenenfalls spült.

**16.** Mittel gemäß eines jeden der Ansprüche 1 bis 14 zur Anwendung in einer Therapiebehandlung der Alopezie (Kahlköpfigkeit).

**17.** Verwendung des Mittels gemäß eines jeden der Ansprüche 1 bis 14 und 16 zur Herstellung eines Medikaments zur Behandlung der Alopezie (Kahlköpfigkeit).